# EUROPEAN PATENT APPLICATION

(11) **EP 4 353 716 A1**
(43) Date of publication of application: **17.04.2024**
(21) Application number: 22819635.8
(22) Date of filing: 10.06.2022
(51) Int. Cl.: C07D 401/14, C07D 417/14, A61K 31/54

(54) **PYRROLE ACYL PIPERYLHYDRAZINE COMPOUND AND USE THEREOF**

(30) Priority: 11.06.2021 CN 202110656714
(71) Applicant: Institute of Materia Medica, Chinese Academy of Medical Sciences, Beijing 100050 (CN)
(72) Inventor: WU, Song, Beijing 100050 (CN); ZHANG, Wenxuan, Beijing 100050 (CN); ZHAO, Xintong, Beijing 100050 (CN); YANG, Qingyun, Beijing 100050 (CN); FENG, Jing, Beijing 100050 (CN); ZHANG, Jie, Beijing 100050 (CN); ZHANG, Chi, Beijing 100050 (CN); HAN, Zunsheng, Beijing 100050 (CN); LI, Tianlei, Beijing 100050 (CN); XIA, Jie, Beijing 100050 (CN); ZHANG, Kun, Beijing 100050 (CN); LIU, Bo, Beijing 100050 (CN); SHAO, Huihui, Beijing 100050 (CN); WANG, Yue, Beijing 100050 (CN); HU, Yuhua, Beijing 100050 (CN); LUO, Xinyu, Beijing 100050 (CN); ZHANG, Hanyilan, Beijing 100050 (CN); LIAN, Xu, Beijing 100050 (CN); ZHU, Zihao, Beijing 100050 (CN)
(74) Representative: Ipside
(86) International application number: PCT/CN2022/098033
(87) International publication number: WO 2022/258038

(57) **Abstract**

The present invention belongs to the technical field of antibacterial drugs, and discloses a pyrrolylacylpiperidylamine compound and use thereof. The present invention in particular relates to a pyrrolylacylpiperidylamine compound and a pharmaceutically acceptable salt thereof, and use thereof in the preparation of a medicament for resisting infections with bacteria, mycoplasma or chlamydia. The compound has a structure shown in the following general formula:

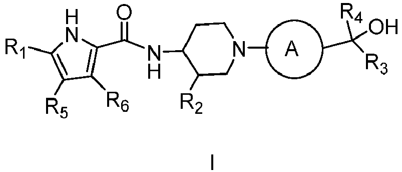

## Description

### TECHNICAL FIELD

The present invention belongs to in the technical field of antibacterial drugs, and in particular relates to a pyrrolylacylpiperidylamine compound and a pharmaceutically acceptable salt, and use thereof as a drug for resisting infections with bacteria, mycoplasma or chlamydia.

### BACKGROUND

Bacterial infections are infections caused by pathogenic or conditionally pathogenic bacteria invading a human body to grow and reproduce, producing toxins and other metabolic products. The bacterial infections are one of the major factors endangering human health, and the emerging bacterial resistance in recent years has made global public health face more severe challenges. Currently, super-resistant bacteria "ESCAPE" include: Enterococcus faecium, Staphylococcus aureus, Clostridium difficile, Acinetobacter baumannii, Pseudomonas aeruginosa and Enterobacteriaceae, which have posed a serious threat to human health. With the abuse of antibiotics and the evolution of bacteria themselves, there are no drugs available for these superbacteria in clinical practice. Therefore, it is extremely urgent to research and develop antibacterial drugs with a new mechanism of action.

Gyr B/Par E dual-target inhibitors of Gyr B subunits of DNA gyrase and Par E subunits of topoisomerase IV have the following advantages in antibacterial drug development: (1) two targets of bacteria can be inhibited simultaneously, with a lower risk of drug resistance; (2) the Gyr B/Par E dual-target inhibitors have a bactericidal mechanism; and (3) the antibacterial effect of a Gyr B/Par E inhibitor novobiocin has undergone historical clinical validation. Therefore, it is feasible to develop antibacterial drugs for this target. However, the development of such Gyr B/Par E inhibitors as antibacterial drugs also has certain problems: 1. most compounds have good enzyme inhibition activity, but have no antibacterial activity in vitro; 2. most compounds do not show in vivo antibacterial activity, or have poor pharmacokinetic properties; and 3. the vast majority of compounds only inhibit the growth of Gram-positive bacteria, but have no or poor effect on Gram-negative bacteria. Due to the above reasons, no Gyr B/Par E dual-target inhibitor drug has been successfully marketed at present, except for novobiocin (which was delisted after marketing). The structural optimization of Gyr B/Par E inhibitors currently under research or the development of new structural types of Gyr B/Par E inhibitors is expected to improve the druggability potential of this target.

DS2969 is a Gyr B/Par E dual-target inhibitor developed by Daiichi Sankyo Co, has good antibacterial activity and druggability, is currently under phase I clinical study, and is mainly used for the treatment of infections caused by Clostridium difficile (WO2017056012). However, DS2969 still has certain disadvantages, such as the need to further improve an activity against positive bacteria in vitro (Staphylococcus aureus ATCC 29213, MIC=0.2 µg/mL), and weak activity against Gram-negative bacteria (Escherichia coli ATCC25922, MIC=32 µg/mL). In addition, AZD5099, developed by AstraZeneca (J. Med. Chem. 57, 6060-6082), has also entered clinical research, but has a higher coefficient of variation of exposure in vivo, a risk of mitochondrial damage, and weak activity against Gram-negative bacteria (Escherichia coli ATCC25922, MIC=32 µg/mL), and was finally withdrawn from clinical trials.

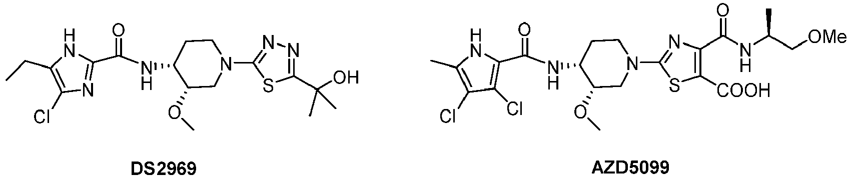

### SUMMARY

An object of the present invention is to overcome the disadvantages of the Gyr B/Par E inhibitors in the prior art, and to provide a broad-spectrum pyrrolylacylpiperidylamine compound having more potent antibacterial, antimycoplasmal or antichlamydial activity and druggability, and a pharmaceutically acceptable salt thereof, and use thereof as a drug for resisting infections with bacteria/mycoplasma/chlamydia.

The present invention provides the following technical solutions:
according to a first aspect of the present invention, provided is a pyrrolylacylpiperidylamine compound and a pharmaceutically acceptable salt or stereoisomer thereof, having a structure shown in a general formula I: 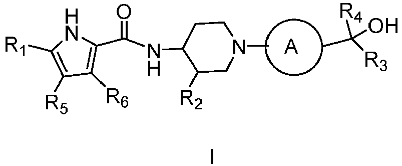
wherein R₁, R₅, and R₆ are each independently selected from H, halogen, hydroxy, amino, nitro, cyano, carboxy, C₁₋₆ alkyl, C₁₋₆ alkoxy, C₁₋₆ alkylamino, di(C₁₋₆ alkyl)amino, hydroxyC₁₋₆ alkyl, aminoC₁₋₆ alkyl, haloC₁₋₆ alkyl or haloC₁₋₆ alkoxy;
R₂ is selected from H, halogen, hydroxy, amino, C₁₋₆ alkyl, C₁₋₆ alkoxy, C₁₋₆ alkylthio, haloC₁₋₆ alkyl, haloC₁₋₆ alkoxy, haloC₁₋₆ alkylthio, C₁₋₆ alkylamino, di(C₁₋₆ alkyl)amino, aminoC₁₋₆ alkoxy, and aminoC₁₋₆ cycloalkoxy;
R₃ and R₄ are each independently selected from H, C₁₋₆ alkyl, and haloC₁₋₆ alkyl; or R₃ and R₄ form C₃₋₈ cycloalkyl or C₃₋₈ heterocycloalkyl together with a carbon atom to which they are connected;
A is selected from phenyl or 5- to 8-membered heteroaryl optionally substituted with 1-4 Q1; and
Q1 is selected from H, halogen, hydroxy, amino, nitro, cyano, carboxy, C₁₋₆ alkyl, C₁₋₆ alkoxy, C₁₋₆ alkylthio, C₁₋₆ alkylamino, di(C₁₋₆ alkyl)amino, hydroxyC₁₋₆ alkyl, aminoC₁₋₆ alkyl, carboxyC₁₋₆ alkyl, haloC₁₋₆ alkyl, haloC₁₋₆ alkoxy or haloC₁₋₆ alkylthio.

Further, the pyrrolylacylpiperidylamine compound and the pharmaceutically acceptable salt or stereoisomer thereof have a structure shown in a general formula IA, 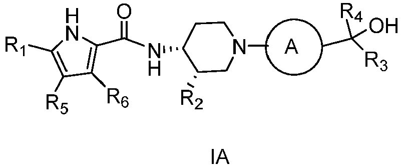
wherein R₁, R₅, and R₆ are each independently selected from H, halogen, hydroxy, amino, C₁₋₆ alkyl, C₁₋₆ alkoxy, haloC₁₋₆ alkyl or haloC₁₋₆ alkoxy;
R₂ is selected from H, halogen, hydroxy, amino, C₁₋₆ alkyl, C₁₋₆ alkoxy, C₁₋₆ alkylthio, haloC₁₋₆ alkyl, haloC₁₋₆ alkoxy, haloC₁₋₆ alkylthio, C₁₋₆ alkylamino, di(C₁₋₆ alkyl)amino, aminoC₁₋₆ alkoxy, and aminoC₁₋₆ cycloalkoxy;
R₃ and R₄ are each independently selected from H, C₁₋₆ alkyl, and haloC₁₋₆ alkyl; or R₃ and R₄ form C₃₋₈ cycloalkyl or C₃₋₈ heterocycloalkyl together with a carbon atom to which they are connected;
A is selected from 5- to 6-membered heteroaryl optionally substituted with 1-3 Q1; and
Q1 is selected from H, halogen, hydroxy, amino, nitro, cyano, carboxy, C₁₋₆ alkyl, C₁₋₆ alkoxy, C₁₋₆ alkylthio, C₁₋₆ alkylamino, di(C₁₋₆ alkyl)amino, hydroxyC₁₋₆ alkyl, aminoC₁₋₆ alkyl, carboxyC₁₋₆ alkyl, haloC₁₋₆ alkyl, haloC₁₋₆ alkoxy or haloC₁₋₆ alkylthio.

Still further, A is selected from thiazole, furan, thiophene, pyrazole, imidazole, oxazole, thiadiazole, pyridine, pyrazine, pyrimidine or pyridazine;
Still further, R₁, R₅, and R₆ are each independently selected from H, fluorine, chlorine, bromine, hydroxy, amino, methyl, ethyl, propyl, isopropyl, butyl, isobutyl, sec-butyl, tert-butyl, methoxy, ethoxy, propoxy, isopropoxy, trifluoromethyl, trifluoroethyl, trifluoropropyl, trifluoromethoxy or trifluoroethoxy;
R₂ is selected from H, fluorine, chlorine, bromine, hydroxy, amino, methyl, ethyl, propyl, isopropyl, butyl, isobutyl, sec-butyl, tert-butyl, methoxy, ethoxy, propoxy, isopropoxy, methylthio, ethylthio, propylthio, trifluoromethyl, trifluoroethyl, trifluoropropyl, trifluoromethoxy, trifluoroethoxy, trifluoromethylthio or trifluoroethylthio, methylamino, dimethylamino, aminoethoxy, aminopropoxy, and aminocyclopentyloxy;
R₃ and R₄ are each independently selected from H, methyl, ethyl, propyl, isopropyl, butyl, isobutyl, sec-butyl, tert-butyl, trifluoromethyl, trifluoroethyl or trifluoropropyl; or, R₃ and R₄ form cyclopropyl, cyclobutyl, cyclopentyl, cyclohexyl, cycloheptyl, cyclooctyl, oxiranyl, oxetanyl, aziridinyl, azetidinyl, 1,4-dioxanyl, 1,3-dioxanyl, 1,3-dioxolanyl, tetrahydrofuranyl, tetrahydropyrrolidinyl, tetrahydropyrazolidinyl, tetrahydroimidazolidinyl, tetrahydrothienyl, tetrahydrothiazolidinyl, tetrahydropyranyl, tetrahydrothiopyranyl, piperidinyl, hexahydropyridyl, piperazinyl or morpholinyl together with a carbon atom to which they are connected; and
A is selected from furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, pyrimidinyl, pyridazinyl, pyrazinyl, 1,2,3-triazinyl, 1,3,5-triazinyl or 1,2,4,5-tetrazinyl.

Preferably, the compound has a structure shown in a general formula IAa, 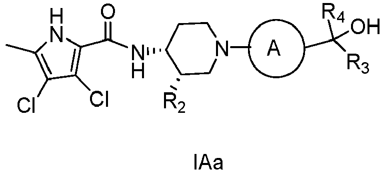
wherein R₂ is selected from H, fluorine, chlorine, bromine, hydroxy, amino, methyl, ethyl, propyl, isopropyl, butyl, isobutyl, sec-butyl, tert-butyl, methoxy, ethoxy, propoxy, isopropoxy, methylthio, ethylthio, propylthio, trifluoromethyl, trifluoroethyl, trifluoropropyl, trifluoromethoxy, trifluoroethoxy, trifluoromethylthio or trifluoroethylthio, methylamino, dimethylamino, aminoethoxy, aminopropoxy, and aminocyclopentyloxy;
R₃ and R₄ are each independently selected from H, methyl, ethyl, propyl, isopropyl, butyl, isobutyl, sec-butyl, tert-butyl, trifluoromethyl, trifluoroethyl or trifluoropropyl; or, R₃ and R₄ form cyclopropyl, cyclobutyl, cyclopentyl or cyclohexyl together with a carbon atom to which they are connected; and
A is selected from furyl, thienyl, pyrrolyl, thiazolyl, isothiazolyl, thiadiazolyl, oxazolyl, isoxazolyl, oxadiazolyl, imidazolyl, pyrazolyl, 1,3,4-oxadiazolyl, pyridyl, pyrimidinyl, pyridazinyl, pyrazinyl, 1,2,3-triazinyl, 1,3,5-triazinyl or 1,2,4,5-tetrazinyl.

Most preferably, the compound has the following structure:

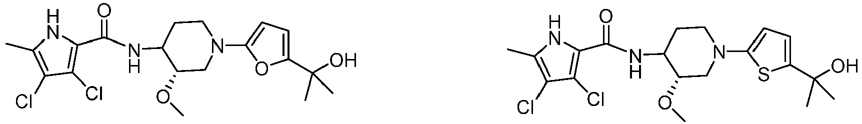

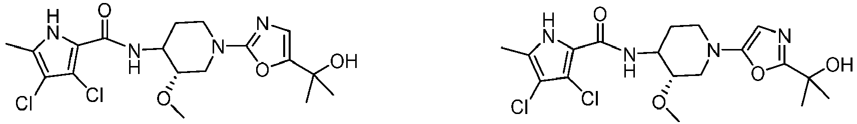

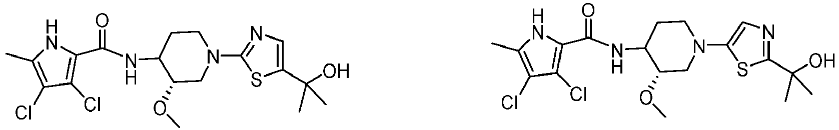

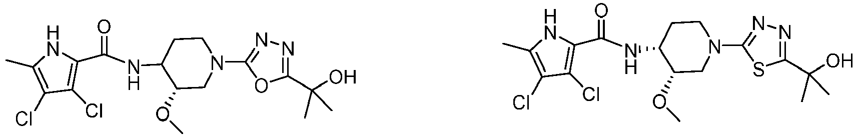

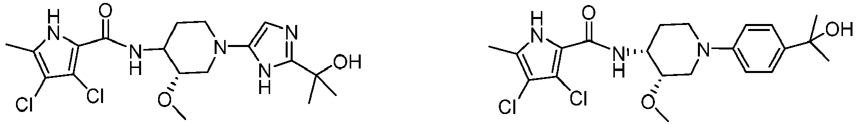

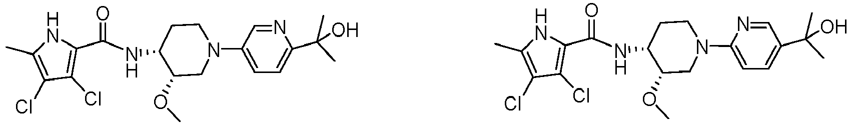

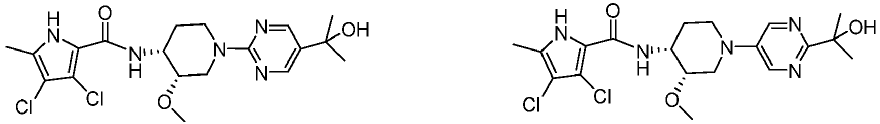

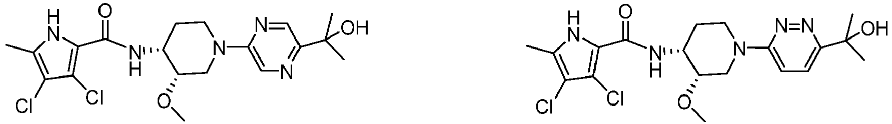

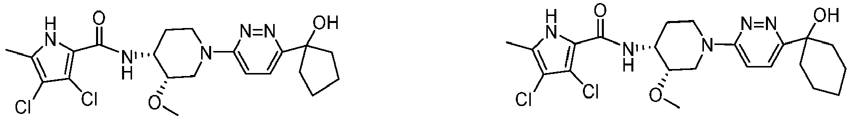

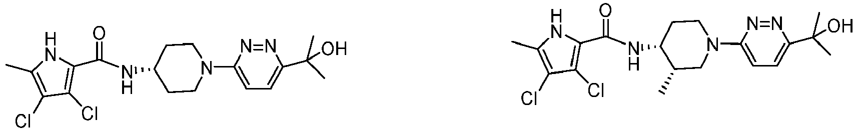

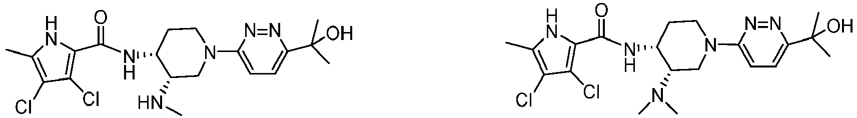

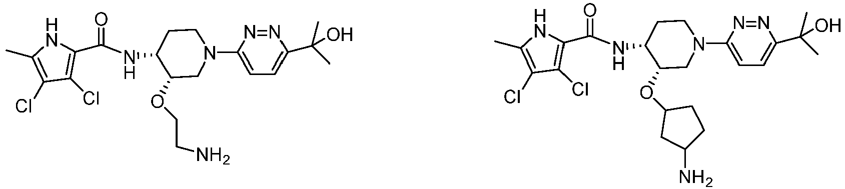

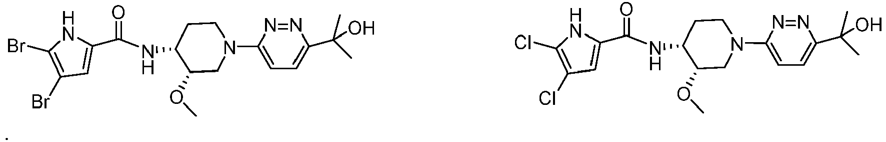

According to a second aspect of the present invention, provided is a pharmaceutical composition, including the pyrrolylacylpiperidylamine compound and the pharmaceutically acceptable salt thereof in the first aspect, and a pharmaceutically acceptable carrier or excipient, further including one or more other active ingredients.

The compound of the present invention or the pharmaceutical composition containing the compound may be administered in a unit dosage form by enteral or parenteral routes such as oral administration, intravenous injection, intramuscular injection, subcutaneous injection, intranasal administration, oral mucosal administration, ocular administration, lung administration and respiratory tract administration, dermal administration, vaginal administration, rectal administration or the like.

An administration dosage form may be a liquid dosage form, a solid dosage form or a semi-solid dosage form. The liquid dosage form may be a solution (including a true solution and a colloidal solution), an emulsion (including an o/w emulsion, a w/o emulsion and a double emulsion), a suspension, an injection (including an aqueous injection, a powder injection and infusion), an eye drop, a nose drop, a lotion, a liniment or the like; the solid dosage form may be a tablet (including a conventional tablet, an enteric tablet, a buccal tablet, a dispersible tablet, a chewable tablet, an effervescent tablet, and an orally disintegrating tablet), a capsule (including a hard capsule, a soft capsule, and an enteric capsule), a granule, a powder, a pellet, a dripping pill, a suppository, a film, a patch, an aerosol (a powder aerosol), a spray or the like; and the semi-solid dosage form may be an ointment, a gel, a paste, or the like.

The compound of the present invention may be formulated into an ordinary formulation, as well as a sustained release formulation, a controlled release formulation, a targeted formulation, and various particulate delivery systems.

For formulating the compound of the present invention into tablets, various excipients well known in the art can be widely used, including a diluent, a binder, a wetting agent, a disintegrant, a lubricant, and a cosolvent. The diluent can be starch, dextrin, sucrose, glucose, lactose, mannitol, sorbitol, xylitol, microcrystalline cellulose, calcium sulfate, dibasic calcium phosphate, calcium carbonate, or the like; the wetting agent can be water, ethanol, isopropanol, or the like; the binder may be starch slurry, dextrin, syrup, honey, a glucose solution, microcrystalline cellulose, acacia mucilage, gelatin paste, sodium carboxymethylcellulose, methylcellulose, hydroxypropylmethylcellulose, ethylcellulose, acrylic resin, carbomer, polyvinylpyrrolidone, polyethylene glycol or the like; the disintegrant can be dry starch, microcrystalline cellulose, low-substituted hydroxypropyl cellulose, cross-linked polyvinylpyrrolidone, cross-linked sodium carboxymethylcellulose, sodium carboxymethyl starch, sodium bicarbonate and citric acid, polyoxyethylene sorbitol fatty acid ester, sodium dodecyl sulfonate or the like; and the lubricant and the cosolvent can be talc, silica, stearate, tartaric acid, liquid paraffin, polyethylene glycol or the like.

The tablets may also be further formulated into coated tablets, such as sugar-coated tablets, film-coated tablets, enteric-coated tablets, or double-layer tablets and multi-layered tablets.

In order to formulate an administration unit into a capsule, an active ingredient, namely the compound of the present invention may be mixed with a diluent and a cosolvent, and the mixture is placed directly into a hard or soft capsule. The active ingredient, namely the compound of the present invention may also be formulated into granules or pellets with a diluent, a binder, and a disintegrant, and the granules or pellets are then placed in a hard or soft capsule. The various diluents, binders, wetting agents, disintegrants, and cosolvents used in the preparation of the tablets of the compound of the present invention may also be used in the preparation of capsules of the compound of the present invention.

In order to formulate the compound of the present invention into an injection, water, ethanol, isopropanol, propylene glycol or a mixture thereof can be used as a solvent, and appropriate amounts of a solubilizer, a cosolvent, a pH adjuster, and an osmotic pressure adjuster commonly used in the art are added. The solubilizer or cosolvent may be poloxamer, lecithin, hydroxypropyl-beta-cyclodextrin, or the like; the pH adjuster can be phosphate, acetate, hydrochloric acid, sodium hydroxide, or the like; and the osmotic pressure adjuster can be sodium chloride, mannitol, glucose, phosphate, acetate, or the like. For example, mannitol, glucose or the like may also be added as a proppant to prepare a freeze-dried powder injection.

In addition, a colorant, a preservative, a flavor, a flavoring agent or other additives may also be added to a pharmaceutical formulation, if desired.

The medicament or pharmaceutical composition of the present invention may be administered by any of the well-known methods of administration in order to achieve the purpose of administration and enhance the therapeutic effect.

The administration dosage of the compound and the pharmaceutical composition of the present invention can vary widely depending upon the nature and severity of a disease to be prevented or treated, the individual condition of a patient or animal, a route of administration and a dosage form, and the like. In general, a suitable daily dose of the compound of the present invention ranges from 0.1 mg/Kg body weight to 50 mg/Kg body weight. The above dose may be administered in one dosage unit or several dosage units, depending on the clinical experience of a physician and an administration regimen selected from other treatment methods. The compound or composition of the present invention may be administered alone or in combination with other therapeutic or symptomatic drugs. When the compound of the present invention has a synergistic effect with other therapeutic drugs, its dosage should be adjusted according to the actual situation.

According to a third aspect of the present invention, provided is use of the pyrrolylacylpiperidylamine compound and the pharmaceutically acceptable salt or stereoisomer thereof, the pharmaceutical formulation and the pharmaceutical composition in the preparation of an antibacterial, antimycoplasmal or antichlamydial medicament. The susceptible or drug-resistant Gram-positive bacteria are selected from Bacillus subtilis, Staphylococcus aureus, Staphylococcus epidermidis, Enterococcus faecalis, Enterococcus faecium, Clostridium difficile, Streptococcus, Diplococcus pneumoniae, Bacillus anthracis, Bacillus diphtheriae, and Bacillus tetani, the susceptible or drug-resistant Gram-negative bacteria are selected from Escherichia coli, Acinetobacter baumannii, Pseudomonas aeruginosa, Klebsiella pneumoniae, dysentery bacillus, Bacillus typhi, Proteus, Vibrio cholerae, Neisseria, Shigella spp., Bacillus pneumoniae, Brucella, Bordetella pertussis, and Bacillus influenzae, the mycobacteria are selected from Mycobacterium tuberculosis, Mycobacterium bovis, and Mycobacterium leprae, the mycoplasma are selected from Mycoplasma pneumoniae, Ureaplasma urealyticum, Mycoplasma hominis, and Mycoplasma genitalium, and the Chlamydia are selected from Chlamydia pneumoniae, Chlamydia psittaci, Chlamydia trachomatis and Chlamydia bovis.

According to a fourth aspect of the present invention, provided is a preparation method for the pyrrolylacylpiperidylamine compound and the pharmaceutically acceptable salt or stereoisomer thereof in the first aspect. Pyrrolic acid a is used as a starting material to be first subjected to condensation with tert-butoxycarbonyl protected 4-aminopiperidine (b), followed by deprotection to obtain an intermediate d. The intermediate is then coupled with bromoheterocycle (e) to obtain an intermediate f, and the intermediate f is finally converted by a Grignard reaction or the like to obtain a target product. For the synthesis of the stereoisomer, a chiral compound b fragment can be used for preparation.

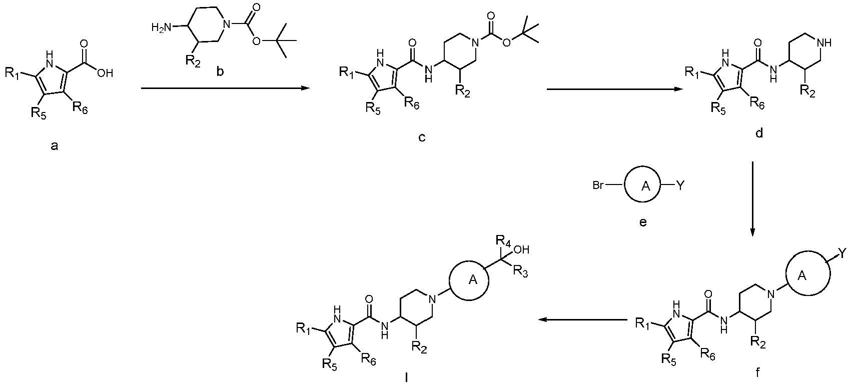

### Terms:

In the present invention, the term "alkyl" refers to a group consisting of carbon and hydrogen atoms and having a specified number of carbon atoms, which may be linear or branched alkyl, "aminoalkyl" refers to alkyl substituted with amino, and "haloalkyl" refers to alkyl substituted with halogen. "Alkoxy" refers to a group formed by linking alkyl to an oxygen atom. "Alkylamino" refers to a group formed by linking alkyl to a nitrogen atom, and "dialkylamino" refers to a group formed by separately linking two alkyl to a nitrogen atom. "Alkylthio" refers to a group formed by linking alkyl to a sulfur atom.

### Beneficial technical effects:

The present invention provides the pyrrolylacylpiperidylamine compound, which has excellent inhibitory effects on Gram-positive bacteria and Gram-negative bacteria, mycobacteria, mycoplasma and chlamydia. Compared with DS2969, the compound has significantly increased inhibitory activity against Gram-positive bacteria, Gram-negative bacteria, mycobacteria, mycoplasma and chlamydia, representing that the activity of the compound against Staphylococcus aureus, Staphylococcus epidermidis, Enterococcus faecalis and Enterococcus faecium is improved by 15 times or more compared with the positive control drug DS2969. At the same time, the compound shows more significant pharmacological activity than the positive control drug DS2969 in a sepsis model infected by drug-resistant bacteria in mice. Compared with the positive control drugs DS2969 and AZD5099, the inhibitory activity of the compound against Escherichia coli is improved by 32 times, the inhibitory activity of the compound against Pseudomonas aeruginosa is improved by at least 8 times or more, and the inhibitory activity of the compound against Acinetobacter baumannii and klebsiella pneumoniae is improved by at least 4 times or more.

### BRIEF DESCRIPTION OF THE DRAWINGS

FIG. 1 is a compound induced drug resistance curve
FIG. 2 is a survival curve (1) for drug efficacy in mice
FIG. 3 is a survival curve (2) for drug efficacy in mice
FIG. 4 is a drug concentration-time curve for intragastric administration in mice
FIG. 5 is a survival curve for acute toxicity test in mice

### DETAILED DESCRIPTION

The present invention is further described by the following examples, however, the scope of the present invention is not limited to the following examples. It can be understood by those skilled in the art that various changes and modifications may be made to the present invention without departing from the spirit and scope of the present invention.

### Example 1: Synthesis of 3,4-dichloro-N-{(3S,4R)-1-[6-(2-hydroxypropan-2-yl)pyridazin-3-yl]-3-methoxypiper idin-4-yl}-5-methyl-1H-pyrrole-2-carboxamide (Compound 1)

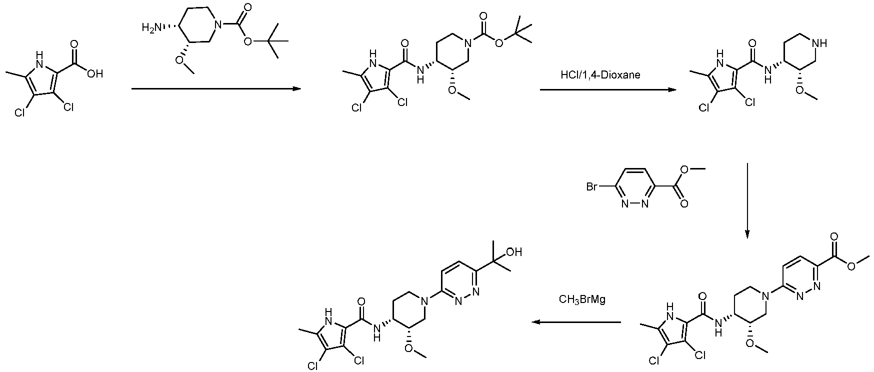

3,4-Dichloro-5-methyl-1H-pyrrole-2-carboxylic acid (1 g, 5.2 mmol) was dissolved in about 6 ml of DMSO, HATU (3 g, 7.7 mmol) and DIPEA (1.3 ml, 7.7 mmol) were added, the mixture was stirred at room temperature for 30 min, tert-butyl (3S,4R)-4-amino-3-methoxypiperidine-1-carboxylate (1.4 g, 6.2 mmol) was added, and the mixture was stirred at room temperature. LC-MS monitoring was performed, after completion of the reaction, an appropriate amount of water was added to the resulting reaction solution to separate out a precipitate, filtering was performed, and a filter cake was sequentially washed with a 0.1 M HCl solution and a saturated NaHCO₃ solution to obtain a compound tert-butyl (3S,4R)-4-(3,4-dichloro-5-methyl-1H-pyrrole-2-carboxamido)-3-methoxypiperidine-1 -carboxylate as white solid (yield: 85%).

The compound tert-butyl (3S,4R)-4-(3,4-dichloro-5-methyl-1H-pyrrole-2-carboxamido)-3-methoxypiperidine-1 -carboxylate (1 g, 2.5 mmol) was dissolved in 1,4-dioxane (12 ml), 6 ml of 4M HCl/a 1,4-dioxane solution was added, the mixture was stirred at 50°C, LC-MS monitoring was performed, and after completion of the reaction, a reaction solvent was distilled off under reduced pressure to obtain a compound 3,4-dichloro-N-((3S,4R)-3-methoxypiperidin-4-yl)-5-methyl-1H-pyrrole-2-carboxami de, which was used directly in a next step.

The compound 3,4-dichloro-N-((3S,4R)-3-methoxypiperidin-4-yl)-5-methyl-1H-pyrrole-2-carboxami de (800 mg, 2.7 mmol) was dissolved in DMF (8 ml), a compound methyl 6-bromopyridazine-3-carboxylate (700 mg, 3.2 mmol) and DIPEA (1.1 ml, 6.8 mmol) were added, and the mixture was stirred at 60°C. LC-MS monitoring was performed, after completion of the reaction, an appropriate amount of water was added to the resulting reaction solution to separate out a precipitate, and filtering and drying were performed to obtain a compound methyl 6-((3S,4R)-4-(3,4-dichloro-5-methyl-1H-pyrrole-2-carboxamido)-3-methoxypiperidin -1-yl)pyridazine-3-carboxylate as a light brown solid (yield: 71%).

The compound methyl 6-((3S,4R)-4-(3,4-dichloro-5-methyl-1H-pyrrole-2-carboxamido)-3-methoxypiperidin -1-yl)pyridazine-3-carboxylate (400 mg, 0.91 mmol) was dissolved in ultra dry tetrahydrofuran (5 ml), a solution (3M) of CH₃BrMg in diethyl ether (1.7 ml, 4.6 mmol) was added dropwise at -5°C under the nitrogen protection, and after the dropwise addition was complete, the mixture was stirred at room temperature. After completion of the reaction, the reaction was quenched with a saturated ammonium chloride solution, and the resulting reaction solution was extracted for three times with ethyl acetate, and EA layers were mixed, washed with saturated brine, dried over anhydrous sodium sulfate, evaporated to dryness under reduced pressure, and subjected to column chromatography (dichloromethane:methanol=20:1) to give a compound 3,4-dichloro-N- { (3S,4R)-1-[6-(2-hydroxypropan-2-yl)pyridazin-3-yl]-3-methoxypiper idin-4-yl}-5-methyl-1H-pyrrole-2-carboxamide as a white solid (yield: 46%).

ESI-MS (m/z): 442.13 [M+H]⁺. ¹H NMR (500 MHz, Chloroform-d) δ 9.58 (s, 1H), 7.36 (d, J = 9.6 Hz, 1H), 6.99 (d, J = 9.6 Hz, 1H), 4.91 (dd, J = 15.0, 2.8 Hz, 1H), 4.35 (dq, J = 6.4, 3.1 Hz, 2H), 4.20 (d, J = 13.6 Hz, 1H), 3.60-3.54 (m, 1H), 3.44 (s, 3H), 3.18-3.08 (m, 2H), 2.28 (s, 3H), 2.00-1.90 (m, 2H), 1.57 (s, 6H).

### Example 2:

### 3,4-Dichloro-N-((3S,4R)-1-(5-(2-hydroxypropan-2-yl)-1,3,4-thiadiazol-2-yl)-3-metho xypiperidin-4-yl)-5-methyl-1H-pyrrole-2-carboxamide (Compound 2)

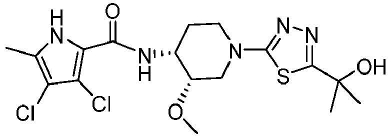

According to a method similar to that in Example 1, the compound 3,4-dichloro-N-((3S,4R)-3-methoxypiperidin-4-yl)-5-methyl-1H-pyrrole-2-carboxami de (Example 1) reacted with 5-bromo-thiadiazole-2-carboxylate, the obtained product was then subjected to a Grignard reaction with CH₃BrMg, and the obtained product was purified to give a target product with a yield of 70%.

ESI-MS (m/z): 448.36 [M+H]⁺. ¹H NMR (400 MHz, Chloroform-d) δ 9.87 (s, 1H), 4.43 (ddd, J = 14.5, 3.2, 2.1 Hz, 1H), 4.36-4.23 (m, 1H), 3.83 (d, J = 13.5 Hz, 1H), 3.52 (q, J = 3.2 Hz, 1H), 3.47 (s, 3H), 3.29 (ddd, J = 13.4, 12.1, 3.1 Hz, 1H), 3.19 (dd, J = 14.4, 1.7 Hz, 1H), 2.88 (s, 1H), 2.28 (s, 3H), 2.12-1.96 (m, 1H), 1.94-1.83 (m, 1H), 1.67 (s, 6H).

### Example 3:

### 3,4-Dichloro-N-((3S,4R)-1-(5-(2-hydroxypropan-2-yl)pyrimidin-2-yl)-3-methoxypipe ridin-4-yl)-5-methyl-1H-pyrrole-2-carboxamide (Compound 3)

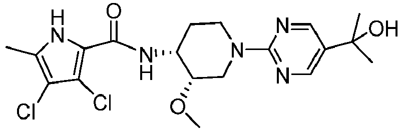

According to a method similar to that in Example 1, the compound 3,4-dichloro-N-((3S,4R)-3-methoxypiperidin-4-yl)-5-methyl-1H-pyrrole-2-carboxami de (Example 1) reacted with 2-bromopyrimidine-2-carboxylate, the obtained product was then subjected to a Grignard reaction with CH₃BrMg, and the obtained product was purified to give a target product with a yield of 62%.

ESI-MS (m/z): 442.34 [M+H]⁺. ¹H NMR (500 MHz, Chloroform-d) δ 9.92 (s, 1H), 8.43 (s, 2H), 7.28 (d, J = 8.2 Hz, 1H), 5.16 (d, J = 14.8 Hz, 1H), 4.77 (d, J = 14.9 Hz, 1H), 4.32 (d, J = 8.2 Hz, 1H), 3.48 (s, 1H), 3.43 (s, 3H), 3.01 (d, J = 13.9 Hz, 2H), 2.28 (s, 3H), 1.86 (d, J = 9.5 Hz, 2H), 1.57 (s, 6H).

### Example 4:

### 3,4-Dichloro-N-((3S,4R)-1-(5-(2-hydroxypropan-2-yl)pyrazin-2-yl)-3-methoxypiperi din-4-yl)-5-methyl-1H-pyrrole-2-carboxamide (Compound 4)

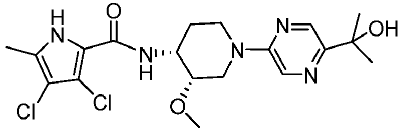

According to a method similar to that in Example 1, the compound 3,4-dichloro-N-((3S,4R)-3-methoxypiperidin-4-yl)-5-methyl-1H-pyrrole-2-carboxami de (Example 1) reacted with 5-bromopyrazine-2-carboxylate, the obtained product was then subjected to a Grignard reaction with CH₃BrMg, and the obtained product was purified to give a target product with a yield of 65%.

ESI-MS (m/z): 442.34 [M+H]⁺. ¹H NMR (500 MHz, Chloroform-d) δ 9.57 (s, 1H), 8.20 (s, 1H), 8.06 (s, 1H), 4.83-4.69 (m, 1H), 4.33 (s, 1H), 4.22 (d, J = 13.5 Hz, 1H), 3.57-3.48 (m, 1H), 3.43 (s, 3H), 3.09 (d, J = 10.3 Hz, 1H), 3.03 (d, J = 15.4 Hz, 1H), 2.28 (s, 3H), 1.93 (d, J = 13.0 Hz, 2H).

### Example 5:

### 3,4-Dichloro-N-((3S,4R)-1-(5-(2-hydroxypropan-2-yl)oxazol-2-yl)-3-methoxypiperidi n-4-yl)-5-methyl-1H-pyrrole-2-carboxamide (Compound 5)

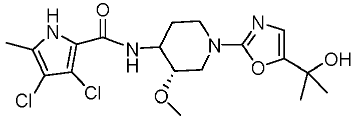

According to a method similar to that in Example 1, the compound 3,4-dichloro-N-((3S,4R)-3-methoxypiperidin-4-yl)-5-methyl-1H-pyrrole-2-carboxami de (Example 1) reacted with 2-bromooxazole-5-carboxylate, the obtained product was then subjected to a Grignard reaction with CH₃BrMg, and the obtained product was purified to give a target product with a yield of 52%.

ESI-MS (m/z): 431.31 [M+H]⁺. ¹H NMR (400 MHz, Chloroform-d) δ 9.35 (s, 1H), 7.22 (d, J = 8.7 Hz, 1H), 6.99 (s, 1H), 4.41 (d, J = 14.3 Hz, 1H), 4.32-4.20 (m, 1H), 4.10 (d, J = 13.1 Hz, 1H), 3.45 (s, 4H), 3.14-2.98 (m, 2H), 2.28 (s, 3H), 1.94 (qd, J = 12.3, 11.7, 4.5 Hz, 1H), 1.82 (dd, J = 13.0, 4.5 Hz, 1H), 1.50 (s, 6H).

### Example 6:

### 3,4-Dichloro-N-((3S,4R)-1-(5-(2-hydroxypropan-2-yl)thiazol-2-yl)-3-methoxypiperid in-4-yl)-5-methyl-1H-pyrrole-2-carboxamide (Compound 6)

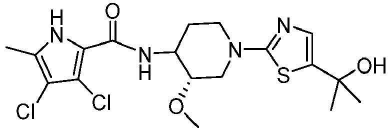

According to a method similar to that in Example 1, the compound 3,4-dichloro-N-((3S,4R)-3-methoxypiperidin-4-yl)-5-methyl-1H-pyrrole-2-carboxami de (Example 1) reacted with 2-bromothiazole-5-carboxylate, the obtained product was then subjected to a Grignard reaction with CH₃BrMg, and the obtained product was purified to give a target product with a yield of 50%.

ESI-MS (m/z): 447.38 [M+H]⁺. ¹H NMR (500 MHz, Chloroform-d) δ 10.03-9.97 (m, 1H), 7.28 (s, 1H), 4.94 (s, 1H), 4.80 (s, 1H), 4.39 (dd, J = 27.6, 13.9 Hz, 1H), 4.29 (s, 1H), 3.90 (t, J = 16.0 Hz, 1H), 3.51 (s, 1H), 3.46 (s, 3H), 3.15 (dt, J = 24.3, 13.8 Hz, 2H), 2.28 (s, 3H), 2.11-1.95 (m, 3H), 1.91-1.81 (m, 1H), 1.28 (d, J = 31.3 Hz, 3H).

### Example 7:

### 3,4-Dichloro-N-((3S,4R)-1-(6-(1-hydroxycyclopentyl)pyridazin-3-yl)-3-methoxypiper idin-4-yl)-5-methyl-1H-pyrrole-2-carboxamide (Compound 7)

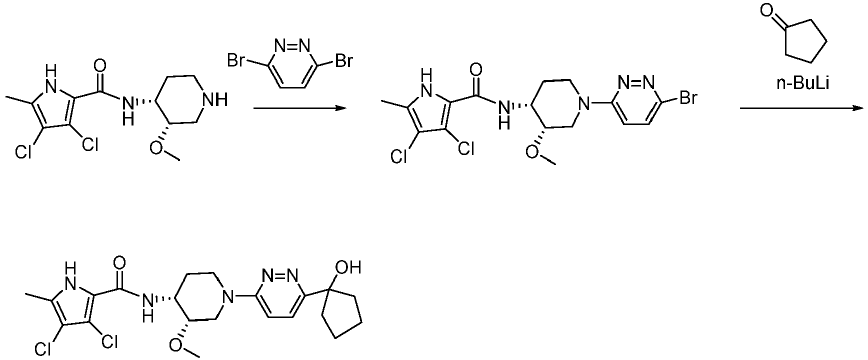

The compound 3,4-dichloro-N-((3S,4R)-3-methoxypiperidin-4-yl)-5-methyl-1H-pyrrole-2-carboxami de (Example 1) reacted with 3,6-dibromopyridazine, the obtained product then reacted with cyclopentanone in the presence of n-butyllithium, and the obtained product was purified to give a target product with a yield of 54%.

ESI-MS (m/z): 468.38 [M+H]⁺. ¹H NMR (500 MHz, Chloroform-d) δ 9.80 (s, 1H), 8.68-8.40 (m, 1H), 7.24 - 7.18 (m, 1H), 6.94 (d, J = 10.2 Hz, 1H), 5.07-4.93 (m, 1H), 4.34 (d, J = 11.3 Hz, 1H), 4.25-4.13 (m, 1H), 3.55 (s, 1H), 3.43 (s, 3H), 3.19-3.03 (m, 2H), 2.28 (s, 3H), 1.85 (d, J = 74.1 Hz, 4H), 1.25 (s, 4H).

### Example 8:

### 3,4-Dichloro-N-((3S,4R)-1-(6-(1-hydroxycyclohexyl)pyridazin-3-yl)-3-methoxypiperi din-4-yl)-5-methyl-1H-pyrrole-2-carboxamide (Compound 8)

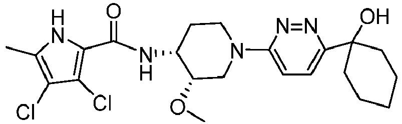

According to a method similar to that in Example 7, the compound 3,4-dichloro-N-((3S,4R)-3-methoxypiperidin-4-yl)-5-methyl-1H-pyrrole-2-carboxami de (Example 1) reacted with 3,6-dibromopyridazine, the obtained product then reacted with cyclohexanone in the presence of n-butyllithium, and the obtained product was purified to give a target product with a yield of 64%.

ESI-MS (m/z): 482.41 [M+H]⁺. ¹H NMR (500 MHz, Chloroform-d) δ 10.11 (s, 1H), 7.30 (d, J = 8.2 Hz, 1H), 7.08 (d, J = 9.3 Hz, 1H), 6.90 (d, J = 9.3 Hz, 1H), 4.93 (dt, J = 14.5, 2.9 Hz, 1H), 4.33 (ddt, J = 11.7, 8.6, 4.2 Hz, 1H), 4.16-4.07 (m, 1H), 3.53 (s, 1H), 3.43 (s, 3H), 3.15-3.03 (m, 2H), 2.82 (dd, J = 8.5, 6.7 Hz, 2H), 2.28 (s, 3H), 2.04-1.87 (m, 2H), 1.70 (p, J = 7.7 Hz, 2H), 1.40 (dd, J = 15.1, 7.6 Hz, 2H).

### Experimental example 1 Evaluation of antibacterial activity in vitro

**Test method:** the in vitro antibacterial activity of the compounds against susceptible/drug-resistant strains was evaluated by microbroth dilution. The in vitro antibacterial activity of the compounds against different strains was tested, and specific operation steps were as follows: a sterilized 96-well plate was placed in a super clean bench, 200 µL of a diluted bacterial solution was added in a first column, and 100 µL of the bacterial solution was added in each column from a second column. 4 µL of a 5 mg/mL test solution in DMSO was sequentially added to each well in the first column, while setting a blank control group (no sample) and a positive drug group (levofloxacin at a concentration of 5 mg/mL), each sample in triplicate. After the sample addition, 128 µL of the sample was sequentially taken from a previous column to be added into a next column by using an 8-channel micropipettor for 2-fold gradient dilution, when the sample was added to the last well, 100 µL of liquid was pipetted after the sample added was uniformly mixed, and the concentration of the compound in each well after dilution was 128, 64, 32, 16, 8, 4, 2, 1, 0.5, 0.25, 0.125, 0.06, 0.03, 0.015, and 0.008 µg/mL, respectively. The 96-well plate was incubated in a 37°C incubator for 18h, and the growth of bacteria in each well was observed. For each compound, the concentration of the compound corresponding to the first well where no bacterial growth was seen by counting backwards from the well into which the sample was added was the Minimal Inhibitory Concentration (MIC) of the compound.

Results: as shown in Table 1, Table 2 and Table 3 below, the compounds showed strong in vitro antibacterial activity, wherein the activity of compounds 1 and 2 against Gram-positive bacteria was improved by 15 times or more compared with DS2969, and was stronger than that of the control drug levofloxacin; the activity against Gram-negative bacteria was improved by at least 4 times or more, with a maximum of 32 times compared with DS2969 and AZD5099; and the activity against clostridium difficile was stronger than those of DS2969 and the positive control drug vancomycin.

**Table 1 In vitro antibacterial activity test of Compounds (MIC, µg/mL)**

| **Compound no.** | Staphylococcus aureus ATCC 29213 | Staphylococcus aureus ATCC 25923 | Staphylococcus aureus ATCC 43300 (MRSA) | Staphylococcus aureus MRSA 15-1 | Staphylococcus epidermidis ATCC 12228 | Staphylococcus epidermidis MRSE 15-1 | Enterococcus faecalis ATCC 51575 | Enterococcus faecium EFM 19-1 |
|---|---|---|---|---|---|---|---|---|
| **1** | 0.008 | ≤0.03 | ≤0.03 | ≤0.03 | ≤0.03 | ≤0.03 | ≤0.03 | ≤0.03 |
| **2** | 0.008 | ≤0.03 | ≤0.03 | ≤0.03 | ≤0.03 | ≤0.03 | ≤0.03 | ≤0.03 |
| **4** | 0.25 | Not tested | Not tested | Not tested | 0.5 | Not tested | Not tested | Not tested |
| **6** | 0.25 | Not tested | Not tested | Not tested | 0.25 | Not tested | Not tested | Not tested |
| **7** | ≤0.03 | Not tested | Not tested | Not tested | 0.125 | Not tested | Not tested | Not tested |
| **8** | ≤0.03 | Not tested | Not tested | Not tested | ≤0.03 | Not tested | Not tested | Not tested |
| **DS2969** | 0.5 | 0.5 | 0.5 | 0.5 | 0.5 | 0.5 | 0.5 | 0.5 |
| **AZD5099** | 0.03 | Not tested | Not tested | Not tested | Not tested | Not tested | Not tested | Not tested |
| **Levofloxacin** | 0.25 | 0.25 | 0.25 | **16** | 0.125 | **4** | 0.5 | 0.5 |

**Table 2 In vitro antibacterial activity test of Compounds (MIC, µg/mL)**

| **Compound no.** | Escherichia coli ATCC 25922 | Escherichia coli ATCC 35218 | Escherichia coli ECO+ 15-1 | Acinetobacter baumannii ATCC 19606 | Pseudomonas aeruginosa ATCC 27853 | Klebsiella pneumoniae KPN-15-1 | Clostridium difficile ATCC 9689 |
|---|---|---|---|---|---|---|---|
| **1** | **1** | **1** | 8 | 8 | 4 | 8 | 0.25 |
| **2** | **2** | **1** | 16 | 16 | 8 | 16 | 0.25 |
| **7** | 8 | Not tested | Not tested | Not tested | Not tested | Not tested | Not tested |
| **8** | 16 | Not tested | Not tested | Not tested | Not tested | Not tested | Not tested |
| **DS2969** | 32 | 32 | >64 | >64 | >64 | >64 | 0.5 |
| **AZD5099** | 32 | 32 | >64 | >64 | >64 | >64 | Not tested |
| **Levofloxacin** | ≤0.06 | ≤0.06 | **8** | 0.25 | 2 | 0.5 | Not tested |
| **Vancomycin** | >64 | >64 | >64 | >64 | >64 | >64 | 2 |

**Table 3 Test for the activity of compounds against Mycobacterium tuberculosis (MIC, µg/mL)**

| **Compound no.** | **Inhibitory activity** |
|---|---|
| **1** | 0.593 |
| **2** | 0.595 |
| **DS2969** | 0.654 |

### Experimental example 2 Evaluation of in vitro induced drug resistance of compound 1

Test method: MIC values of a compound 1 and a control compound DS2969 against Staphylococcus aureus ATCC 29213 were determined by using a microbroth dilution method recommended by operating procedures for antibacterial susceptibility testing of the Clinical and Laboratory Standards Institute (CLSI).

After testing the MIC values, sub-MIC concentrations (1/2 MIC) of bacteria were diluted, and the concentration of the obtained bacterial solution was adjusted to 10⁵ CFU/mL for a next round of MIC value testing. After 24 hours of incubation, bacterial suspensions were prepared by using bacterial suspensions at sub-MIC concentrations of drugs (1/2 MIC), and another MIC testing was performed. This process was repeated for 20 times, and the MIC value for each test was recorded.

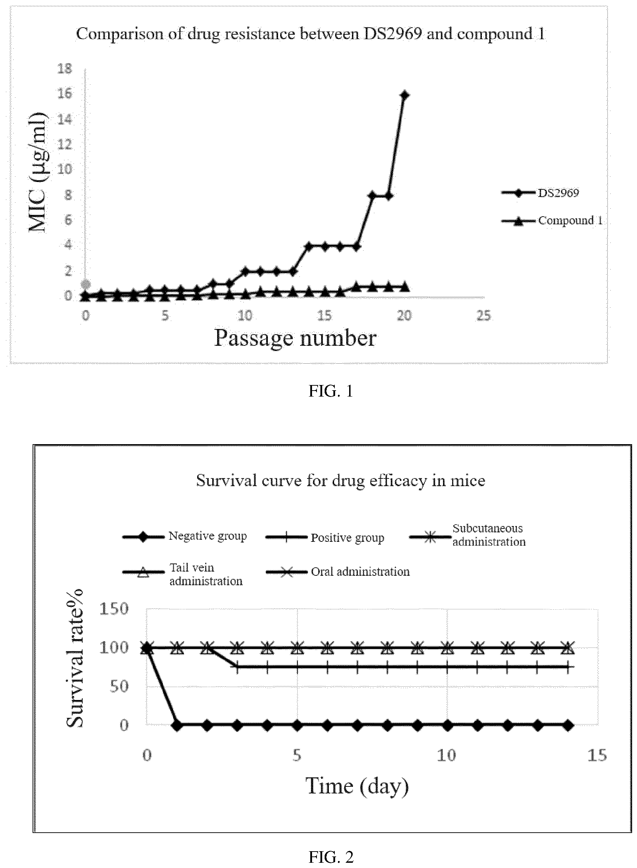

The MIC values of the compound 1 and DS2969 inducing Staphylococcus aureus ATCC29213 for 20 generations were recorded, with the number of generations of bacteria induced as an abscissa and the MIC values as an ordinate (as shown in FIG. 1).

Results: after passage for 20 generations, the MIC value of the compound 1 was less than 1.0 µg/mL, but the MIC value of the positive control drug DS2969 was 16 µg/mL.

### Experimental example 3 Evaluation of antibacterial activity in mice

Experimental animals: Beijing Vital River Laboratory Animal Technology Co., Ltd., ICR mice, with a uniform body weight of 20-22 g, and half male and half female.

Model: Sepsis model infected by Staphylococcus aureus (MRSA, ATCC 43300)
Modeling method: mice were infected with 2×10⁷-8×10⁶ CFU/mL (diluted with a 5% sterile yeast solution) Staphylococcus aureus (MRSA, ATCC 43300) such that sepsis models of mice were formed (0.5 mL of a bacterial solution was injected intraperitoneally into each mouse).

Experimental scheme: a compound 1 and a positive drug were first dissolved in DMSO, and then diluted with other solvents, wherein a solvent ratio was saline:Tween 80:DMSO=9:0.5:0.5. The mice were divided into 5 groups, 4 mice in each group, half female and half male, and an administration group was administered with 0.4 mL of a 2.5 mg/mL compound solution at a dose of 50 mg/kg by intravenous injection, subcutaneous injection and intragastric administration at 1h after the modeling, a negative control group was injected with 0.4 mL of a blank solvent after the modeling, and a positive control group was injected with a positive drug DS2969 subcutaneously at the same dose as the compound group, and a survival rate of the mice was observed for 14 days.

Results: as shown in FIG. 2, the compound 1 has significant in vivo antibacterial activity and shows better antibacterial activity than DS2969.

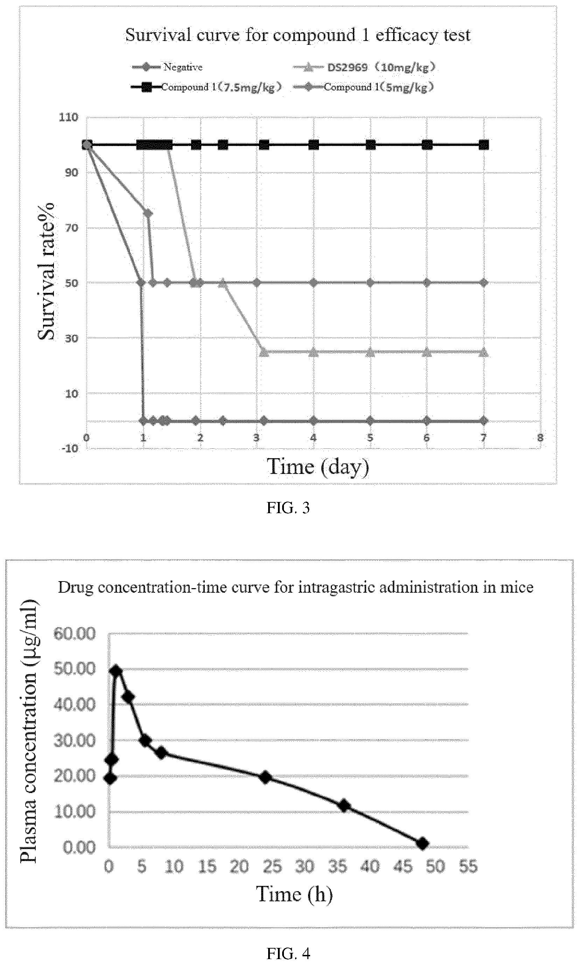

In the same model, the efficacy of the compound 1 administered by intragastric administration at a lower dose was further examined, and the results are shown in FIG. 3, which shows that ED₅₀ of the compound 1 is 5 mg/kg, while ED50 of DS2969 is more than 10 mg/kg.

### Experimental example 4 Pharmacokinetic test in mice

Experimental animals: Beijing Vital River Laboratory Animal Technology Co., Ltd., ICR mice, with a uniform body weight of 20-22 g, and half male and half female.

Experimental scheme: a compound 1 was administered at a dose of 150 mg/kg (Solvents were as follows: saline:Tween 80:DMSO=9:0.5:0.5) by intragastric administration, and samples were taken by orbital blood sampling or a mode of sampling blood by enucleating eyeballs at 10 min, 20 min, 30 min, 1h, 3h, 5h, 8h, 24h, 36h, and 48h after administration, plasma concentrations were determined by UPLC, a drug concentration-time curve was plotted, and key pharmacokinetic parameters were calculated.

Results: the drug concentration-time curve is shown in FIG. 4, key pharmacokinetic parameters are shown in Table 3, a half-life period is 9.2 h, and bioavailability is 86%, indicating that the plasma concentration of the compound 1 in vivo can be maintained for a long time, and that the compound 1 can be well absorbed in vivo, providing a good support for the therapeutic effect.

**Table 4 Pharmacokinetic parameters of compound 1 administered by intragastric administration**

| Parameter | Unit | Numerical value |
|---|---|---|
| AUC (0-48) | mg/L*h | 916.416 |
| AUC (0-∞) | mg/L*h | 933.251 |
| MRT (0-48) | h | 16.094 |
| MRT (0-∞) | h | 16.907 |
| t1/2z | h | 9.208 |
| Tmax | h | 1 |
| Cmax | mg/L | 49.42 |
| F | % | 86 |

### Experimental example 5 Acute toxicity test in mice

Experimental animals: Beijing Vital River Laboratory Animal Technology Co., Ltd., ICR mice, with a uniform body weight of 20-22 g, and half male and half female.

Experimental scheme: a compound 1 (solvents were as follows: saline:Tween 80:DMSO=9:0.5:0.5) was administrated by tail vein injection, intraperitoneal administration and intragastric administration, respectively, and a survival rate of the mice was observed for 14 days. In a tail vein administration group, there were 10 mice in each group, with half male and half female. In an intragastric administration group and an intraperitoneal administration group, there were four mice in each group, with half male and half female.

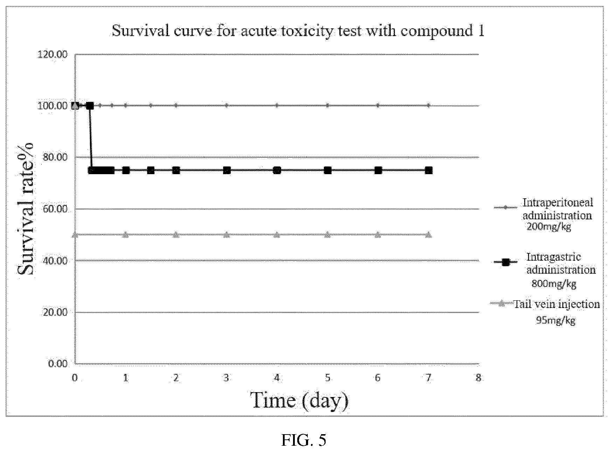

Results: as shown in FIG. 5, the survival rate was 60% on day 14 in a group administered with 95 mg/kg by a tail vein, the survival rate was 100% on day 14 in a group administered with 200 mg/kg intraperitoneally, and the survival rate was 75% on day 14 in a group administered with 800 mg/kg by intragastric administration.

## Claims

1. A pyrrolylacylpiperidylamine compound and a pharmaceutically acceptable salt or stereoisomer thereof, **characterized by** having a structure shown in a general formula I, 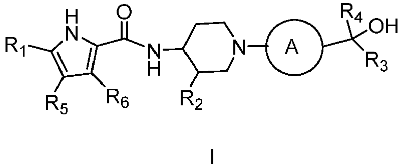
wherein R₁, R₅, and R₆ are each independently selected from H, halogen, hydroxy, amino, nitro, cyano, carboxy, C₁₋₆ alkyl, C₁₋₆ alkoxy, C₁₋₆ alkylamino, di(C₁₋₆ alkyl)amino, hydroxyC₁₋₆ alkyl, aminoC₁₋₆ alkyl, haloC₁₋₆ alkyl or haloC₁₋₆ alkoxy;
R₂ is selected from H, halogen, hydroxy, amino, C₁₋₆ alkyl, C₁₋₆ alkoxy, C₁₋₆ alkylthio, haloC₁₋₆ alkyl, haloC₁₋₆ alkoxy, haloC₁₋₆ alkylthio, C₁₋₆ alkylamino, di(C₁₋₆ alkyl)amino, aminoC₁₋₆ alkoxy, and aminoC₁₋₆ cycloalkoxy;
R₃ and R₄ are each independently selected from H, C₁₋₆ alkyl, and haloC₁₋₆ alkyl; or R₃ and R₄ form C₃₋₈ cycloalkyl or C₃₋₈ heterocycloalkyl together with a carbon atom to which they are connected;
A is selected from phenyl or 5- to 8-membered heteroaryl optionally substituted with 1-4 Q1; and
Q1 is selected from H, halogen, hydroxy, amino, nitro, cyano, carboxy, C₁₋₆ alkyl, C₁₋₆ alkoxy, C₁₋₆ alkylthio, C₁₋₆ alkylamino, di(C₁₋₆ alkyl)amino, hydroxyC₁₋₆ alkyl, aminoC₁₋₆ alkyl, carboxyC₁₋₆ alkyl, haloC₁₋₆ alkyl, haloC₁₋₆ alkoxy or haloC₁₋₆ alkylthio.

2. The pyrrolylacylpiperidylamine compound and the pharmaceutically acceptable salt or stereoisomer thereof according to claim 1, **characterized by** having a structure shown in a general formula IA, 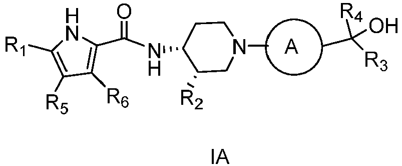
wherein R₁, R₅, and R₆ are each independently selected from H, halogen, hydroxy, amino, C₁₋₆ alkyl, C₁₋₆ alkoxy, haloC₁₋₆ alkyl or haloC₁₋₆ alkoxy;
R₂ is selected from H, halogen, hydroxy, amino, C₁₋₆ alkyl, C₁₋₆ alkoxy, C₁₋₆ alkylthio, haloC₁₋₆ alkyl, haloC₁₋₆ alkoxy, haloC₁₋₆ alkylthio, C₁₋₆ alkylamino, di(C₁₋₆ alkyl)amino, aminoC₁₋₆ alkoxy, and aminoC₁₋₆ cycloalkoxy;
R₃ and R₄ are each independently selected from H, halogen, C₁₋₆ alkyl, and haloC₁₋₆ alkyl; or R₃ and R₄ form C₃₋₈ cycloalkyl or C₃₋₈ heterocycloalkyl together with a carbon atom to which they are connected;
A is selected from 5- to 6-membered heteroaryl optionally substituted with 1-3 Q1; and
Q1 is selected from H, halogen, hydroxy, amino, nitro, cyano, carboxy, C₁₋₆ alkyl, C₁₋₆ alkoxy, C₁₋₆ alkylthio, C₁₋₆ alkylamino, di(C₁₋₆ alkyl)amino, hydroxyC₁₋₆ alkyl, aminoC₁₋₆ alkyl, carboxyC₁₋₆ alkyl, haloC₁₋₆ alkyl, haloC₁₋₆ alkoxy or haloC₁₋₆ alkylthio.

3. The pyrrolylacylpiperidylamine compound and the pharmaceutically acceptable salt or stereoisomer thereof according to any one of claims 1-2, **characterized in that**
A is selected from furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, pyrimidinyl, pyridazinyl, pyrazinyl, 1,2,3-triazinyl, 1,3,5-triazinyl or 1,2,4,5-tetrazinyl optionally substituted with 1-2 Q1.

4. The pyrrolylacylpiperidylamine compound and the pharmaceutically acceptable salt or stereoisomer thereof according to any one of claims 1-2, **characterized in that**
R₁, R₅, and R₆ are each independently selected from H, fluorine, chlorine, bromine, hydroxy, amino, methyl, ethyl, propyl, isopropyl, butyl, isobutyl, sec-butyl, tert-butyl, methoxy, ethoxy, propoxy, isopropoxy, trifluoromethyl, trifluoroethyl, trifluoropropyl, trifluoromethoxy or trifluoroethoxy;
R₂ is selected from H, fluorine, chlorine, bromine, hydroxy, amino, methyl, ethyl, propyl, isopropyl, butyl, isobutyl, sec-butyl, tert-butyl, methoxy, ethoxy, propoxy, isopropoxy, methylthio, ethylthio, propylthio, trifluoromethyl, trifluoroethyl, trifluoropropyl, trifluoromethoxy, trifluoroethoxy, trifluoromethylthio or trifluoroethylthio, methylamino, dimethylamino, aminoethoxy, aminopropoxy, and aminocyclopentyloxy;
R₃ and R₄ are each independently selected from H, methyl, ethyl, propyl, isopropyl, butyl, isobutyl, sec-butyl, tert-butyl, trifluoromethyl, trifluoroethyl or trifluoropropyl;
or, R₃ and R₄ form cyclopropyl, cyclobutyl, cyclopentyl, cyclohexyl, oxiranyl, oxetanyl, aziridinyl, azetidinyl, 1,4-dioxanyl, 1,3-dioxanyl, 1,3-dioxolanyl, tetrahydrofuranyl, tetrahydropyrrolidinyl, tetrahydropyrazolidinyl, tetrahydroimidazolidinyl, tetrahydrothienyl, tetrahydrothiazolidinyl, tetrahydropyranyl, tetrahydrothiopyranyl, piperidinyl, hexahydropyridyl, piperazinyl or morpholinyl together with a carbon atom to which they are connected; and
A is selected from furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, pyrimidinyl, pyridazinyl, pyrazinyl, 1,2,3-triazinyl, 1,3,5-triazinyl or 1,2,4,5-tetrazinyl.

5. The pyrrolylacylpiperidylamine compound and the pharmaceutically acceptable salt thereof according to any one of claims 1-2, **characterized by** having a structure shown in a general formula IAa, 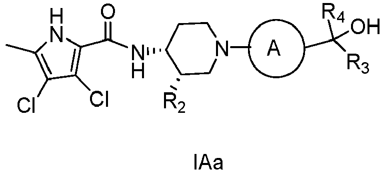
wherein R₂ is selected from H, fluorine, chlorine, bromine, hydroxy, amino, methyl, ethyl, propyl, isopropyl, butyl, isobutyl, sec-butyl, tert-butyl, methoxy, ethoxy, propoxy, isopropoxy, methylthio, ethylthio, propylthio, trifluoromethyl, trifluoroethyl, trifluoropropyl, trifluoromethoxy, trifluoroethoxy, trifluoromethylthio or trifluoroethylthio, methylamino, dimethylamino, aminoethoxy, aminopropoxy, and aminocyclopentyloxy;
R₃ and R₄ are each independently selected from H, methyl, ethyl, propyl, isopropyl, butyl, isobutyl, sec-butyl, tert-butyl, trifluoromethyl, trifluoroethyl or trifluoropropyl;
or, R₃ and R₄ form cyclopropyl, cyclobutyl, cyclopentyl or cyclohexyl together with a carbon atom to which they are connected; and
A is selected from furyl, thienyl, pyrrolyl, thiazolyl, isothiazolyl, thiadiazolyl, oxazolyl, isoxazolyl, oxadiazolyl, imidazolyl, pyrazolyl, 1,3,4-oxadiazolyl, pyridyl, pyrimidinyl, pyridazinyl, pyrazinyl, 1,2,3-triazinyl, 1,3,5-triazinyl or 1,2,4,5-tetrazinyl.

6. The pyrrolylacylpiperidylamine compound and the pharmaceutically acceptable salt thereof according to claim 1, **characterized in that** the compound has the following structure:

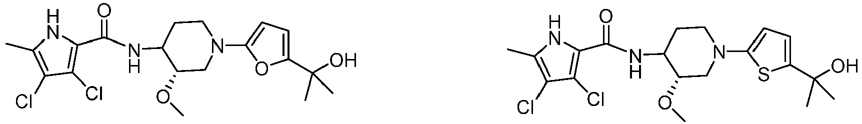

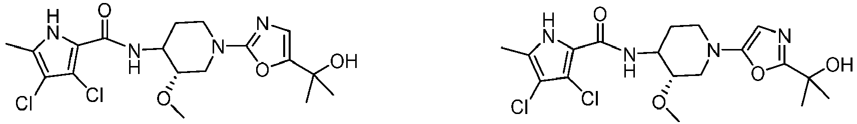

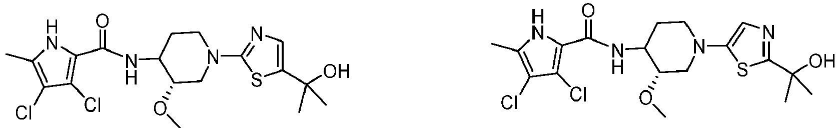

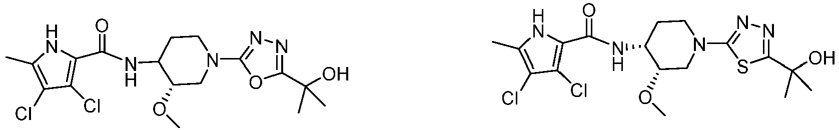

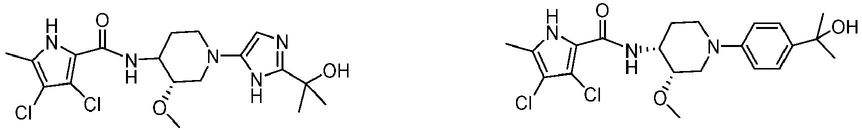

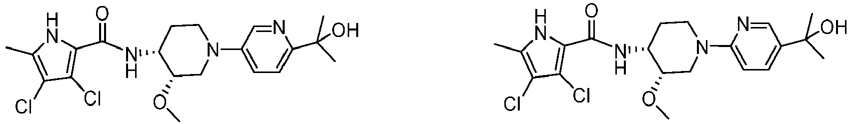

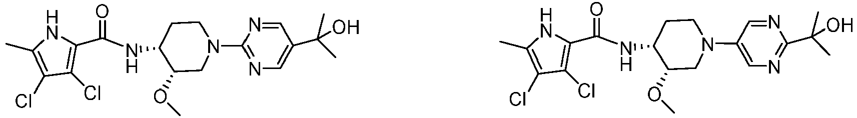

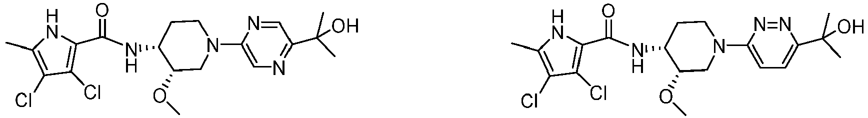

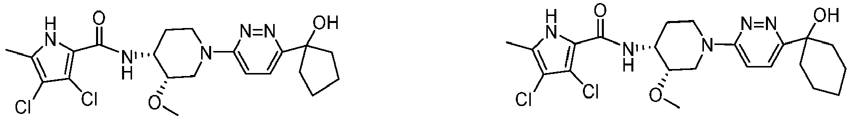

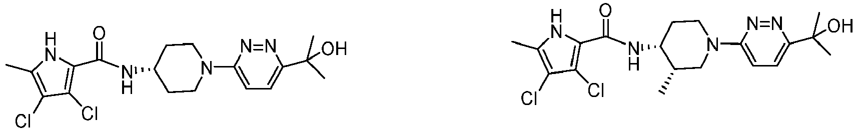

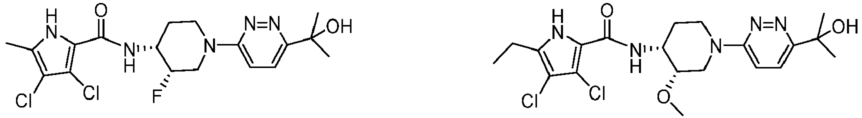

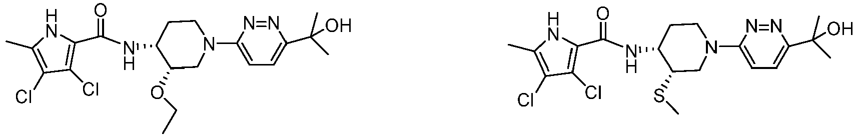

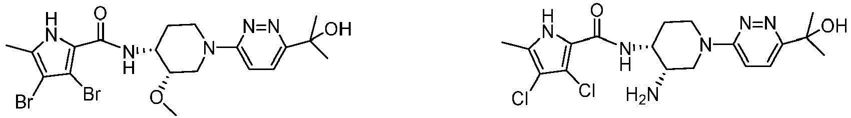

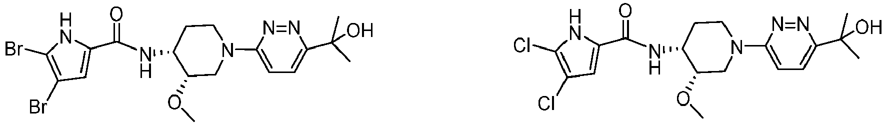

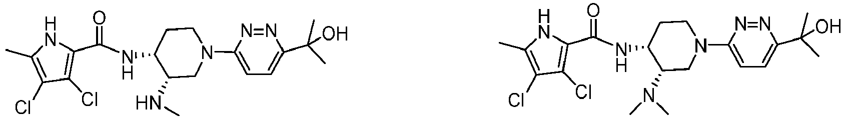

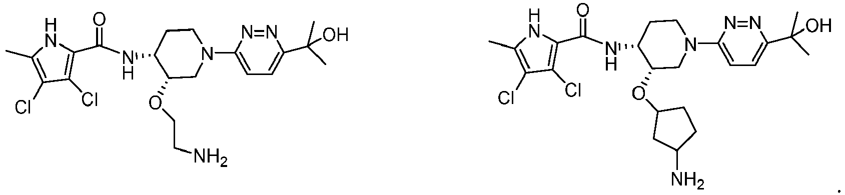

7. A pharmaceutical composition, **characterized by** comprising the pyrrolylacylpiperidylamine compound and the pharmaceutically acceptable salt or stereoisomer thereof according to any one of claims 1-6, and one or more pharmaceutically acceptable carriers or excipients.

8. The pharmaceutical composition according to claim 7, **characterized by** further comprising one or more other pharmacologically active ingredients.

9. Use of the pyrrolylacylpiperidylamine compound and the pharmaceutically acceptable salt or stereoisomer thereof according to any one of claims 1-6, and the pharmaceutical composition according to any one of claims 7-8 in the preparation of a medicament for the treatment of infections with bacteria, mycoplasma or chlamydia.

10. The use according to claim 9, **characterized in that** the bacteria are selected from susceptible or drug-resistant Gram-positive bacteria, Gram-negative bacteria and mycobacteria, the mycoplasma are selected from susceptible or drug-resistant mycoplasma and the chlamydia are selected from susceptible or drug-resistant chlamydia.

11. The use according to claim 10, **characterized in that** the Gram-positive bacteria are selected from Bacillus subtilis, Staphylococcus aureus, Staphylococcus epidermidis, Enterococcus faecalis, Enterococcus faecium, Clostridium difficile, Streptococcus, Diplococcus pneumoniae, Bacillus anthracis, Bacillus diphtheriae, and Bacillus tetani, the Gram-negative bacteria are selected from Escherichia coli, Acinetobacter baumannii, Pseudomonas aeruginosa, Klebsiella pneumoniae, dysentery bacillus, Bacillus typhi, Proteus, Vibrio cholerae, Neisseria, Shigella spp., Bacillus pneumoniae, Brucella, Bordetella pertussis, and Bacillus influenzae, the mycobacteria are selected from Mycobacterium tuberculosis, Mycobacterium bovis, and Mycobacterium leprae, the mycoplasma are selected from Mycoplasma pneumoniae, Ureaplasma urealyticum, Mycoplasma hominis, and Mycoplasma genitalium, and the Chlamydia are selected from Chlamydia pneumoniae, Chlamydia psittaci, Chlamydia trachomatis and Chlamydia bovis.
